# EUROPEAN PATENT SPECIFICATION

(11) **EP 1 852 700 B1**
(45) Date of publication and mention of the grant of the patent: **05.08.2009**
(21) Application number: 06714154.9
(22) Date of filing: 21.02.2006
(51) Int. Cl.: G01N 33/52, G01N 31/22, G01N 21/77, G01N 21/78, C12Q 1/26, C12Q 1/28, C12Q 1/48

(54) **DRY ANALYSIS ELEMENT**
TROCKENANALYSEELEMENT
ELÉMENT D'ANALYSE PAR VOIE SÈCHE

(30) Priority: 28.02.2005 JP 2005052947
(43) Date of publication of application: 07.11.2007
(73) Proprietor: FUJIFILM Corporation, Minato-ku Tokyo 106-0031 (JP)
(72) Inventor: MURAYA, Koji, Asaka-shi, Saitama (JP)
(74) Representative: Albrecht, Thomas
(86) International application number: PCT/JP2006/303014
(87) International publication number: WO 2006/092980

(56) References cited:
- EP-A- 0 333 114
- JP-A- 05 099 927
- JP-A- 06 503 964
- JP-A- 09 184 837
- JP-A- 63 032 499
- JP-A- 2002 369 698
- JP-A- 2003 315 333
- US-A1- 2003 202 904

## Description

### Technical Field

The present invention relates to a dry analysis element capable of determining a biochemical material in a trace amount of a sample solution rapidly / simply.

### Background Art

As the dry analysis elements (including all of a multilayer analysis element, a dry analysis element, and a multilayer analysis device) used for determining biochemical materials, enzymes, and other materials in vivo, there are the technologies described in, for example, Patent Document 1 and Patent Document 2. These relate to the dry analysis element for carrying out the quantitative analysis of the components contained in a biological sample such as blood, lymph, cerebrospinal fluid, or urine.

Whereas, not only for the general biochemical materials, enzymes, or the like in the biological samples, in a very large number of the biosynthesis reactions in vivo or in vitro, release of pyrophosphate and decomposition of pyrophosphate are included. For this reason, when the biosynthesis reaction is studied from the thermodynamical viewpoint, determination of pyrophosphate is effective. For example, the nucleic acid synthesis proceeds while catalyzing the polymerization of deoxynucleoside triphosphate by DNA polymerase. At this step, pyrophosphate is released as the by-product of the nucleic acid synthesis. In the nucleic acid synthesis, the release of pyrophosphate is very important from the viewpoint of energy. Further, it is also important to confirm whether the nucleic acid synthesis was carried out or not by detecting the pyrophosphate. For example, for the reaction solution after the amplification reaction of nucleic acid, the following is also frequently carried out. By carrying out the determination of pyrophosphate, whether amplification was actually carried out or not is confirmed.

The technology of the dry analysis element for use in determination of pyrophosphate in such a liquid sample in vivo or in vitro is described in, for example, Patent Document 3. This is as follows. In a dry analysis element, pyrophosphate (PPi) is converted to inorganic phosphate (Pi) by inorganic pyrophosphatase (PPase). The inorganic phosphate (Pi) is allowed to react with xanthosine or inosine by purine nucleoside phosphorylase (PNP). The resulting xanthine or hypoxanthine is oxidized by xanthine oxidase (XOD) to form uric acid. By the use of hydrogen peroxide (H₂O₂) formed in this oxidation process, a coupler (dye precursor such as a leuco dye) is allowed to develop its color by peroxidase (POD), and this is subjected to colorimetry.

Whereas, as the reaction system for measuring pyrophosphate, the following is also known. At and subsequent to the reaction in which xanthine or hypoxanthine is formed, for example, a coupler (e.g., a formazan type dye such as nitrotetrazolium blue) is allowed to develop its color by NADH formed by the use of xanthine dehydrogenase (XDH), and diaphorase (DI), and this is subjected to colorimetry.

The dry analysis element herein usable is configured such that one layer of, or a plurality of layers of functional layers are provided on a transparent support. A detection reagent is allowed to be contained in at least one layer (or, over a plurality of layers). Thus, after the dropwise addition of a given amount of the liquid sample, the resulting dye formed by the reaction in the layer is subjected to colorimetry by a reflected light or a transmitted light externally of the analysis element.

After the dropwise addition of a given amount of a liquid sample, the dry analysis element is held at a given temperature (incubation), so that the color reaction is allowed to proceed sufficiently. Then, for example, an illumination light is exposed from the transparent support side. Thus, the reflected light amount or the transmitted light amount is measured in a specific wavelength region, so that the reflected light density or the transmitted light density is determined. Then, the quantitative analysis is carried out based on the previously determined calibration curve.

For example, in the dry analysis element for the determination of pyrophosphate described in Patent Document 3, or the dry analysis element described in Patent Document 4, in the related art, other than a layer containing a reagent required for the color reaction such as an enzyme, a coloring material, or a substrate, and the transparent support, there may be provided a development layer for the purpose of holding and rapidly developing the liquid sample which has been added dropwise. In this case, as the development layer, there can be used a membrane filter (brush polymer), a three-dimensional lattice granular structure including polymer beads in contact with each other in a point contact manner through a polymer adhesive which will not swell with water, fabric, or the like.
Patent Document 1: JP-A-60-82859
Patent Document 2: JP-A-60-222770
Patent Document 3: JP-A-2004-156983
Patent Document 4: JP-A-2003-270249

### Disclosure of the Invention

### Problems that the Invention is to Solve

However, when the development layer including the material is provided as in the Patent Documents 3 and 4, and is subjected to transmission photometry, the light incident upon the inside of the dry analysis element is affected by attenuation / scattering, or the like. This may impede the precise measurement. On the other hand, removal of the development layer is advantageous for transmission photometry. However, this time, the liquid permeation rate of the liquid sample added dropwise into the functional layer is slowed, which is not suitable for the rapid quantification.

It is an object of the invention to provide a dry analysis element which is capable of analyzing, for example, a biological material in a liquid sample rapidly and simply, and is not required to be provided with a development layer.

### Means for Solving the Problems

The present inventors conducted a close study in order to solve the foregoing problem. As a result, they found out the following. By allowing a gelatin thin film to contain therein a water absorbing polymer, the permeation rate of a liquid sample into the gelatin thin film is raised even without providing a development layer. Thus, it is possible to provide a dry analysis element capable of implementing a rapid analysis. Thus, the invention was completed.

Namely, the invention is as follows:
(1) A dry analysis element, which comprises, on a transparent support, one reagent layer comprising a gelatin thin film containing at least a polyacrylate type water absorbing polymer, wherein the reagent layer comprises at least one surfactant, wherein the surfactant comprises a compound containing silicon.
(2) The dry analysis element as described in (1) above,
   wherein in the reagent layer, the water absorbing polymer is present in an amount of not more than 10 mass% based on an amount of gelatin per unit area.
(3) The dry analysis element as described in (1) or (2) above,
   wherein a gelatin content in the reagent layer falls within a range of 2 to 100 g/m².
(4) The dry analysis element as described in (4) above,
   wherein in the reagent layer, the surfactant is present in an amount of not more than 80 mass% based on an amount of gelatin per unit area.
(5) The dry analysis element as described in any of (1) to (4) above,
   wherein the reagent layer comprises: a reagent for converting pyrophosphate into inorganic phosphate; and a reagent group for effecting a color reaction according to an amount of the converted inorganic phosphate.
(6) The dry analysis element as described in (5) above,
   wherein the reagent layer comprises: xanthosine or inosine; inorganic pyrophosphatase; purine nucleoside phosphorylase; xanthine oxidase; peroxidase; and a coupler.
(7) The dry analysis element as described in (6) above,
   wherein the coupler is a leuco type dye.
(8) The dry analysis element as described in (5) above,
   wherein the reagent layer comprises: xanthosine or inosine; inorganic pyrophosphatase; purine nucleoside phosphorylase; xanthine dehydrogenase; diaphorase; and a coupler.
(9) The dry analysis element as described in (8) above,
   wherein the coupler is a formazan type dye.
(10) The dry analysis element as described in any of (5) to (9) above,
   wherein the reagent layer comprises magnesium ions.
(11) The dry analysis element as described in any of (1) to (10) above, which is used for carrying out determination of a biological material with colorimetry by reflection photometry or transmission photometry in which a liquid sample containing a biological material is added dropwise on the reagent layer, so as to develop a color.

### Advantage of the Invention

In accordance with the invention, the reagent layer includes a gelatin thin film containing at least a water absorbing polymer. Therefore, a liquid sample rapidly permeates into the gelatin thin film by the action of the water absorbing polymer. As a result of this, it becomes possible to carry out the quantitative analysis of, for example, a biological material in the liquid sample rapidly and simply.

### Best Mode for Carrying Out the Invention

The object to be measured of a dry analysis element in accordance with the present invention has no particular restriction so long as it is a biological material. Examples thereof may include pyrophosphate, glucose, GPT, GOT, uric acid, total cholesterol, neutral fat, and albumin.

When pyrophosphate is the object to be measured, it is possible to use, for example, the reaction principle shown in the following reaction formula 1 or formula 2. In the reaction system shown by the following formula 1 or formula 2, pyrophosphate (PPi) is converted to inorganic phosphate (Pi) by inorganic pyrophosphatase (PPase). The inorganic phosphate (Pi) is allowed to react with xanthosine or inosine by purine nucleoside phosphorylase (PNP). The resulting xanthine or hypoxanthine is oxidized by xanthine oxidase (XOD) to form uric acid. By the use of hydrogen peroxide (H₂O₂) formed in this oxidation process, a coupler is allowed to develop its color by peroxidase (POD), and this is subjected to colorimetry.

Incidentally, as the coupler, N,N-di-substituted aniline such as 4-aminoantipyrin, phenols or naphthols, and a leuco dye are exemplified. Out of these, dye precursor such as a leuco dye is preferred.

Alternatively, as shown in the Formula 3, the following process is also possible. At and subsequent to the reaction in which xanthine or hypoxanthine is formed, for example, a coupler is allowed to develop its color by NADH formed from the reduction of NAD by the use of xanthine dehydrogenase (XDH), and diaphorase (DI), and this is subjected to colorimetry.

Incidentally, as the couplers, formazan type dye is exemplified. Out of these, nitrotetrazolium blue or the like is preferable.

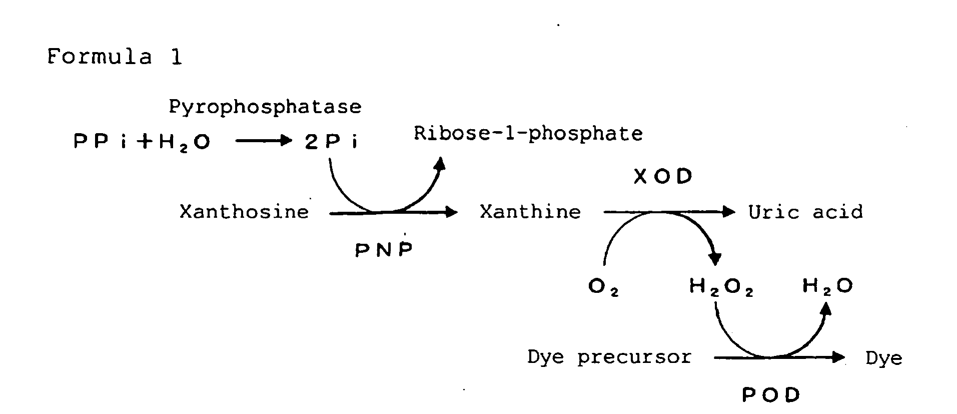

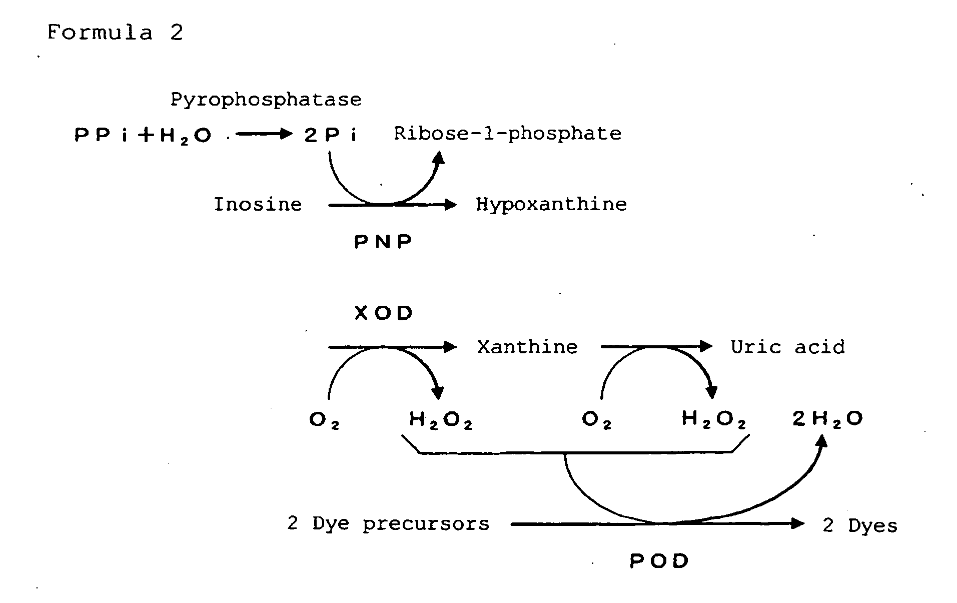

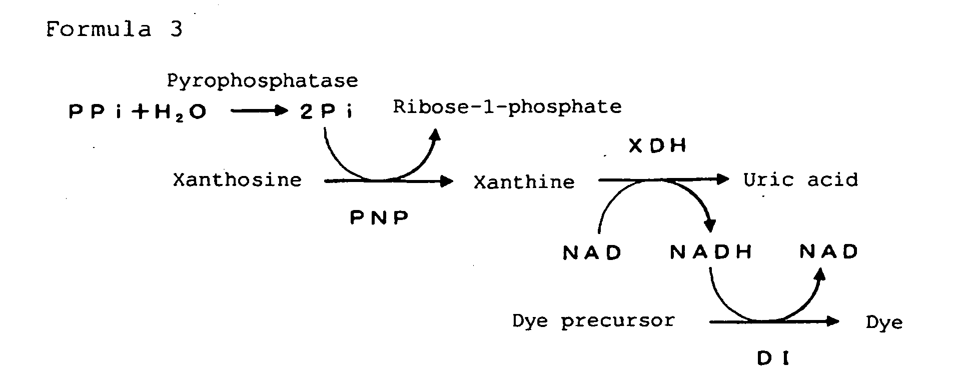

Alternatively, other color reaction for determination of pyrophosphate is also usable. For example, after converting pyrophosphate to inorganic phosphate by inorganic pyrophosphatase, a known color reaction for determination of inorganic phosphate can be used. For the step at and subsequent to the reaction after conversion to inorganic phosphate, as described in the Patent Document 3, use of a known inorganic phosphate determination method in the field of biochemical inspection can effect the color reaction according to the amount of inorganic phosphate.

Also for other biological materials than pyrophosphate, by incorporating a known color reaction for determination of each material into the dry analysis element of the invention, it is possible to carry out determination with simplicity /rapidly. As for the other biological materials than pyrophosphate, the following documents can serve as references.
Glucose: JP-A-1-231897
GPT: JP-A-57-144996
GOT: JP-A-62-195299
Uric acid: JP-A-63-219400
Total cholesterol: JP-A-2-181656
Neutral fat: JP-A-63-119695
Albumin: JP-A-62-137564

For the dry analysis element of the invention, a dry method is used. The element is reserved / stored in dry state until the analysis is carried out. Therefore, a reagent is not required to be prepared before use as with a so-called wet method. Further, generally, the reagent is higher in safety in dry state, and hence, the dry method is superior in simplicity / rapidness to the wet method. Whereas, it is also excellent as a method capable of carrying out a high precision inspection rapidly with a trace amount of a liquid sample.

The dry analysis element of the invention can assume the same layer structure as that of known diverse dry analysis elements. The dry analysis element may be a multilayer including, other than a reagent layer containing a reagent group including reagents such as an enzyme and a substrate for measuring a biological material, a transparent support, a detection layer, an adhesion layer, a water absorption layer, an undercoat layer, a gas permeation layer, a buffer layer, and other layers. As such dry analysis elements, there are the ones disclosed in each specification of, for example, JP-A-49-53888 (corresponding to U.S. Pat. No. 3,992,158), JP-A-51-40191 (corresponding to U.S. Pat. No. 4,402,335), JP-A-55-164356 (corresponding to U.S. Pat. No. 4,292,272), and JP-A-61-4959 (corresponding to EPC No. 0166365A).

The dry analysis element when a light transmissive water impermeable support is used can practically assume the following structure. However, the contents of the invention is not limited thereto.
(1) The one having a reagent layer on a support;
(2) The one having a detection layer and a reagent layer in this order on a support;
(3) The one having a second reagent layer and a first reagent layer in this order on a support; or
(4) The one having a detection layer, a second reagent layer, and a first reagent layer in this order on a support.

As shown in the item (3) or (4), the reagent layer may include a plurality of different layers. For example, when pyrophosphate is the object to be measured, the pyrophosphatase reaction and the PNP reaction may be allowed to proceed at the first reagent layer, and the XOD reaction and the POD reaction may be allowed to proceed at the second reagent layer. Alternatively, the pyrophosphatase reaction, the PNP reaction, and the XOD reaction may be allowed to proceed at the first reagent layer, and the POD reaction may be allowed to proceed at the second reagent layer.

Below, respective layers will be described in details. However, the invention is not limited thereto.

Support: Any material is usable. However, a light transmissive and water impermeable support is preferable. Preferred materials for the light transmissive and water impermeable support are polyethylene terephthalate and polystyrene. In order for the hydrophilic layer to firmly adhere thereto, generally, an undercoat layer is provided on the surface thereof, or a hydrophilization treatment is performed thereon. The thickness of the support has no particular restriction. However, the proper thickness is about 10 to 1000 µm, and preferably 50 to 300 µm.

Detection layer: The detection layer contains at least a coupler. When the object to be measured is pyrophosphate, color development is effected according to the amount of pyrophosphate. The detection layer and the reagent layer may be separated from each other. However, the detection layer and the reagent layer may be integrated. Further, the detection layer may contain, other than the coupler, a reagent group for effecting the color reaction such as an enzyme. The material forming the detection layer has no particular restriction. However, gelatin is preferably suitable. The thickness of the detection layer has no particular restriction. However, when it is provided as a coating layer, the proper thickness is in the range of about 1 to about 500 µm, and preferably about 10 to about 100 µm. In the case of multilayer formation by other method than coating, such as multilayer formation by laminating, the thickness can largely change within the range of several tens of micrometers to several hundreds of micrometers. The amount of gelatin in the detection layer is in the range of 2 to 100 g/m², and preferably 5 to 50 g/m².

Reagent layer: The reagent layer includes a gelatin thin film containing at least a water absorbing polymer. It contains, for example, the foregoing reagent for converting pyrophosphate to inorganic phosphate, and a reagent group for effecting the color reaction according to the amount of the converted inorganic phosphate.

As the gelatin contained in the reagent layer, a derivative (such as phthalated gelatin) is also usable. The thickness of the reagent layer has no particular restriction. However, when it is provided as a coating layer, the proper thickness is in the range of about 1 to about 500 µm, and preferably about 10 to about 100 µm. In the case of multilayer formation by other method than coating, such as multilayer formation by laminating, the thickness can largely change within the range of several tens of micrometers to several hundreds of micrometers.

The amount of gelatin in the reagent layer is in the range of 2 to 100 g/m², and preferably 5 to 50 g/m².

The water absorbing polymer is added to the reagent layer for once holding the liquid sample added dropwise, and then, promptly allowing it to permeate into the reagent layer. For this reason, with the dry analysis element of the invention, even when a development layer in which a liquid sample containing the biological sample as described above is allowed to diffuse / permeate is not provided on the reagent layer, it is possible to carry out a measurement rapidly.
The water absorbing polymer is a polyacrylate type.

As for the particle diameter of the water absorbing polymer, the one with a central particle diameter of about 10 to about 800 µm is preferably used, and the one of about 10 to about 100 µm is more preferred. Further, the water absorbing polymer of the type highly crosslinked by modification or the like, or of the type enhanced in salt resistance is also usable.

The water absorbing polymer can be used in an amount of not more than 10 mass% based on the amount of gelatin per unit area in the reagent layer. Whereas, the amount of the water absorbing polymer to be added is preferably 0.1 mass% or more. The more preferred amount of the water absorbing polymer to be used is 1 to 5 mass% based on the amount of gelatin per unit area.

In order to enhance the effect of the water absorbing polymer in the invention, namely, in order to further enhance the effect of once holding the liquid sample, and then, promptly allowing it to permeate into the reagent layer, a surfactant

is added to the reagent layer. Particularly, a silicon-containing surfactant is used. Further, a nonreactive polyether-modified silicon oil is preferred as the surfactant. The one with a H.L.B. in the range of 4 to 16 is usable. However, the one in the range of 7 to 12 is particularly preferred. The surfactant can be used in an amount of not more than 80 mass% based on the amount of gelatin per unit area in the reagent layer. Whereas, the amount of the surfactant to be added is preferably 1 mass% or more. The more preferred amount of the surfactant to be used is 5 to 50 mass% based on the amount of gelatin per unit area.

Incidentally, the silicon-containing surfactant and other surfactants can also be used in combination.

Whereas, in the invention, the inclusion of magnesium ions in the reagent layer is preferable because it produces the effect that the pyrophosphatase reaction at the first stage after dropwise addition of the sample smoothly proceeds. In order to introduce magnesium ions into the reagent layer, generally, it is essential only that magnesium salt such as MgCl₂ is contained in the reagent layer with water. The content of magnesium ions is preferably 1 to 75 mass%, and more preferably 5 to 55 mass% based on the amount of gelatin per unit area in the reagent layer.

To the reagent layer, for the purpose of improving various performances such as coating characteristics, diffusibility of the diffusible compound, reactivity, and storability, an activator and a stabilizer of an enzyme, a coenzyme, a surfactant, a pH buffer composition, a fine powder, an antioxidant, and in addition, various additives including organic matters or inorganic matters may be added other than the various components. Examples of the buffer which can be contained in the reagent layer include the pH buffer system described in KAGAKU BINRAN KISO compiled by the Chemical Society of Japan, (issued in 1966 by MARUZEN Co., Ltd.), pages 1312 to 1320, Data for Biochemical Research, the second edition, compiled by R. M. C. Dawson et al, (issued in 1969 by Oxford at the Clarendon Press), pages 476 to 508, Biochemistry, 5, pages 467 to 477 (1966), and Analytical Biochemistry, 104, pages 300 to 310, (1980). Specific examples of the pH buffer system include a buffer containing borate; a buffer containing citric acid or citrate; a buffer containing glycine; a buffer containing bicine; a buffer containing HEPES; and a Good's buffer such as a buffer containing MES. Incidentally, a buffer containing phosphate cannot be used for a dry analysis element for detection of pyrophosphate.

The reagent layer can be provided by coating an aqueous solution or an aqueous dispersion containing various reagents in the invention and gelatin on the support or other layers such as the detection layer according to the method described in each specification of JP-B-53-21677 (corresponding to U.S. Pat. No. 3, 992, 158), JP-A-55-164356 (corresponding to U.S. Pat. No. 4,292,272), and JP-A-54-101398 (corresponding to U.S. Pat. No. 4,132,528), and drying it. Incidentally, as described above, the reagent layer may include a plurality of different layers.

Incidentally, the dry analysis element in accordance with the invention may be provided with, or not provided with a development layer. However, no provision of the development layer is desirable because it is advantageous for transmission photometry. When the development layer is provided, the development layer can be formed of a membrane filter (brush polymer), a three dimensional lattice granular structure including polymer beads in contact with each other in a point contact manner through a polymer adhesive not swelling with water, fabric, or the like.

The dry analysis element of the invention can be prepared by the known methods described in the foregoing various patent specifications. The dry analysis element is cut into given small pieces in a square measuring about 5 mm to about 30 mm per side, or in a circle with roughly the same size, or the like. The small piece is stored in the slide frame described in JP-B-57-283331 (corresponding to U.S. Pat. No. 4, 169, 751), JP-UM-A-56-142454 (corresponding to U.S. Pat. No. 4,387,990), JP-A-57-63452, JP-UM-A-58-32350, JP-T-58-501144 (the term "JP-T" as used herein means a published Japanese translation of a PCT patent application) (corresponding to WO83/00391), or the like, and used as a chemical analysis slide. Alternatively, the dry analysis element can be used in such a form as to be held in each well bottom of a commercially available 96-hole plate (or a plate with a larger number of wells than that).

Further, as the dry analysis element of the invention, the one in the form of a slide as described above is preferred. However, it may be in the form of a small piece or a long tape according to the intended purpose. Whereas, for the dry analysis element of the invention, cut pieces thereof may be used as they are. However, they may also be used in such a form as to be stored in a cassette, a magazine, or a vessel with an opening, or they may be attached or stored in an open card.

The dry analysis element of the invention can detect and determine the test subject in a liquid sample by the same operation as the operation described in the various patent specifications. For example, an aqueous liquid sample solution in an amount in the range of about 2 to about 30 µL, and preferably 4 to 15 µL is added dropwise (applied in dots) on the reagent layer. The analysis element applied in dots is subjected to incubation at a constant temperature in the range of about 15 to 45°C, and preferably at a constant temperature in the range of about 20 to 40°C for 1 to 30 minutes. The color development or discoloration in the analysis element is subjected to reflection photometry or transmission photometry from the transparent support side. Thus, the amount of the test subject can be determined from the principle of colorimetry using a previously formed calibration curve. By making constant the amount of the liquid sample to be applied in dots, and the incubation time and temperature, it is possible to carry out quantitative analysis with high precision.

For the measurement operation, it is possible to carry out high precision quantitative analysis with a very easy operation by means of the chemical analysis devices described in JP-A-60-125543, JP-A-60-220862, JP-A-61-294367, JP-A-58-161867 (corresponding to U.S. Pat. No. 4,424,191), and the like, and a commercially available plate reader. Incidentally, it is also acceptable according to the purpose or the required precision that the degree of color development is visually judged to carry out a semi-quantitative measurement.

### Examples

The invention will be described more specifically by way of the following examples. However, the invention is not limited by the following examples.

### (1) Dry analysis element for determination of pyrophosphate

On a 180 µm thick colorless transparent polyethylene terephthalate (PET) smooth film sheet (support) including a gelatin undercoat layer provided thereon, an aqueous solution with the composition (a) described in Table 1 was coated so as to achieve the following coverage, and dried to provide a reagent layer. For comparison, the aqueous solution with the composition (b) described in Table 2 was coated in the same manner, and dried to provide a reagent layer.

**Table 1**

| Composition (a) of reagent layer aqueous solution | |
|---|---|
| Gelatin | 18.9 g/m² |
| p-Nonylphenoxy polyglycidol | 0.5 g/m² |
| (glycidol unit: an average of 10 units contained) | |
| (C₉H₁₉-Ph-O-(CH₂CH(OH)-CH₂-O)₁₀H) | |
| Water absorbing polymer | 0.44 g/m² |
| (AQUALIC CA ML-10 manufactured by NIPPON SHOKUBAI Co., Ltd.) | |
| Inosine | 1.6 g/m² |
| Peroxidase | 15200 U/m² |
| Xanthine oxidase | 4600 U/m² |
| Purine nucleoside phosphorylase | 2300 U/m² |
| Inorganic pyrophosphatase | 500 U/m² |
| Dimedone (5,5-dimethylcyclohexane-1,3-dione) | 0.07 g/m² |
| Leuco dye | 0.28 g/m² |
| (2-(3,5-dimethoxy-4-hydroxyphenyl)-4,5-bis(4-dimethylaminophenyl | |
| imidazole) | |
| MgCl₂·6H₂O | 6.8 g/m² |
| Polyether-modified silicone oil | 8.0 g/m² |
| (KF-618 manufactured by Shin-Etsu Chemical Co., Ltd.) | |
| Water | 97.3 g/m² |
| adjusted to a pH of 6.5 with a dilute NaOH/HCl solution | |

| | |
|---|---|
| Note) AQUALIC CA ML-10 which is a water absorbing polymer is a polyacrylate type water absorbing polymer, and has an average particle diameter of 10 µm. | |

**Table 2**

| Composition (b) of reagent layer aqueous solution | |
|---|---|
| Gelatin | 18.9 g/m² |
| p-Nonylphenoxy polyglycidol | 0.5 g/m² |
| (glycidol unit: an average of 10 units contained) | |
| (C₉H₁₉-Ph-O-(CH₂CH(OH)-CH₂-O)₁₀H) | |
| Inosine | 1.6 g/m² |
| Peroxidase | 15200 U/m² |
| Xanthine oxidase | 4600 U/m² |
| Purine nucleoside phosphorylase | 2300 U/m² |
| Pyrophosphatase | 500 U/m² |
| Dimedone | 0.07 g/m² |
| Leuco dye | 0.28 g/m² |
| (2-(3,5-dimethoxy-4-hydroxyphenyl)-4,5-bis(4-dimethylaminophenyl | |
| imidazole) | |
| MgCl₂·6H₂O | 6.8 g/m² |
| Water | 97.3 g/m² |
| adjusted to a pH of 6.5 with a dilute NaOH/HCl solution | |

These dry analysis elements were cut into circular shapes with a diameter of about 6 mm, and bonded to the bottoms of a commercially available 96-hole plate.

### Measurement Example 1: Relation between the pyrophosphate concentration in a liquid sample and the color development of a dry analysis element for determination of pyrophosphate

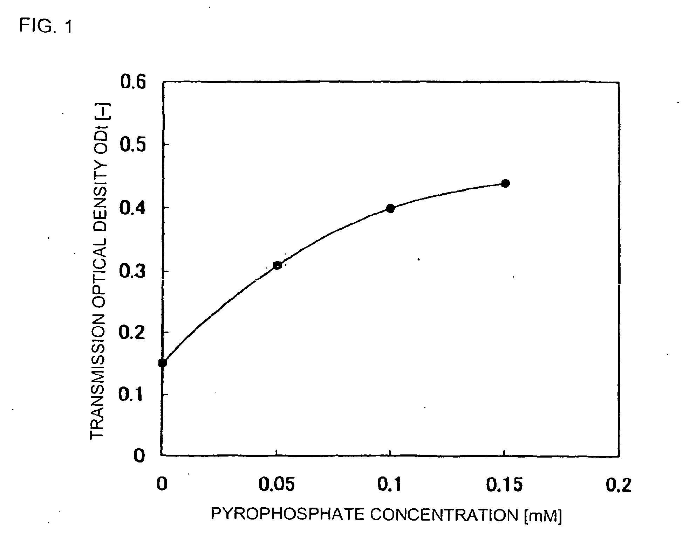

By the use of potassium pyrophosphate with a confirmed purity (manufactured by Wako Pure Chemical Industries Ltd.), pyrophosphate aqueous solutions with respective concentrations of 0; 0.05; 0.10; and 0.15 (mM) were prepared, and applied in an amount of 5 µL in dots on the dry analysis element manufactured in Example. The dry analysis element was incubated at 25 °C for 10 minutes. Then, the transmission density (ODt) was measured from the support side at a wavelength of 650 nm by means of a plate reader (GENios Pro manufactured by TECAN Co.). The results are shown in Table 3 and FIG. 1. This indicates that it is possible to quantitatively measure the pyrophosphate amount in the liquid sample by the use of the dry analysis element.

**Table 3**

| Relation between the pyrophosphate concentration in liquid sample and transmission optical density (ODt) after 10 minutes | |
|---|---|
| | |
| Pyrophosphate concentration (mM) | Transmission optical density (ODt) after 10 minutes |
| 0 | 0.15 |
| 0.05 | 0.31 |
| 0.10 | 0.40 |
| 0.15 | 0.44 |

### Measurement Example 2: Effects of addition of water absorbing polymer and silicon-containing surfactant

For Example of the invention, with the dry analysis elements of the composition (a) and the composition (b) manufactured as a control, the following procedure was carried out. By the use of potassium pyrophosphate with a confirmed purity (manufactured by Wako Pure Chemical Industries Ltd.), pyrophosphate aqueous solutions with respective concentrations of 0; 0.05; and 0.10 (mM) were prepared, and applied in an amount of 5 µL in dots on the dry analysis element manufactured in Example. The dry analysis element was incubated at 25 °C for 10 minutes. Then, the transmission density (ODt) was measured from the support side at a wavelength of 650 nm by means of a plate reader (GENios Pro manufactured by TECAN Co.). The results of the time course of the color reaction are shown as ODt in Table 4. The composition (b) is the one obtained by removing the water absorbing polymer and the silicon-containing surfactant from the composition (a).

The comparison between the composition (a) and the composition (b) indicated as follows. For the composition (b), the noise at 0 minute was high, and further, the permeation was not completed even after an elapse of 5 minutes or more upon application in dots of 5 µL of a test solution (pyrophosphate aqueous solution). In contrast, for the dry analysis element of the composition (a), the permeation was completed within 15 seconds upon application in dots of 5 µL of a test solution. The fact that an increase in permeation rate improves the time course of the color reaction is indicated as the difference in ΔODt between 3 minutes and 10 minutes.

**Table 4**

| Table 4: Color reaction time course upon application in dots of pyrophosphate aqueous solution | | | | |
|---|---|---|---|---|
| (ODt at each measurement time) | | | | |
| Composition (a) | 0 Minute | 3 Minutes | 10 Minutes | ΔODt (10 minutes - 3 minutes) |
| 0.00 mM | 0.069 | 0.122 | 0.151 | 0.029 |
| 0.05 mM | 0.088 | 0.279 | 0.311 | 0.032 |
| 0.10 mM | 0.094 | 0.379 | 0.398 | 0.019 |

| Composition (b) | 0 Minute | 3 Minutes | 10 Minutes | ΔODt (10 minutes - 3 minutes) |
|---|---|---|---|---|
| 0.00 mM | 0.144 | 0.175 | 0.198 | 0.023 |
| 0.05 mM | 0.160 | 0.252 | 0.310 | 0.058 |
| 0.10 mM | 0.197 | 0.339 | 0.396 | 0.057 |

### Brief Description of the Drawing

[FIG. 1] A diagram showing the relationship between the pyrophosphate concentration in a liquid sample and the transmission optical density (ODt) after 10 minutes.

## Claims

1. A dry analysis element, which comprises, on a transparent support, one reagent layer comprising a gelatin thin film containing at least a polyacrylate type water absorbing polymer, wherein the reagent layer comprises at least one surfactant, wherein the surfactant comprises a compound containing silicon.

2. The dry analysis element according to claim 1,
wherein in the reagent layer, the water absorbing polymer is present in an amount of not more than 10 mass% based on an amount of gelatin per unit area.

3. The dry analysis element according to claim 1 or 2,
wherein a gelatin content in the reagent layer falls within a range of 2 to 100 g/m².

4. The dry analysis element according to claim 4,
wherein in the reagent layer, the surfactant is present in an amount of not more than 80 mass% based on an amount of gelatin per unit area.

5. The dry analysis element according to any of claims 1 to 4,
wherein the reagent layer comprises: a reagent for converting pyrophosphate into inorganic phosphate; and a reagent group for effecting a color reaction according to an amount of the converted inorganic phosphate.

6. The dry analysis element according to claim 5,
wherein the reagent layer comprises: xanthosine or inosine; inorganic pyrophosphatase; purine nucleoside phosphorylase; xanthine oxidase; peroxidase; and a coupler.

7. The dry analysis element according to claim 6, wherein the coupler is a leuco type dye.

8. The dry analysis element according to claim 5,
wherein the reagent layer comprises: xanthosine or inosine; inorganic pyrophosphatase; purine nucleoside phosphorylase; xanthine dehydrogenase; diaphorase; and a coupler.

9. The dry analysis element according to claim 8,
wherein the coupler is a formazan type dye.

10. The dry analysis element according to any of claims 5 to 9,
wherein the reagent layer comprises magnesium ions.

11. Use of the dry analysis element according to any of claims 1 to 10 for carrying out determination of a biological material with colorimetry by reflection photometry or transmission photometry in which a liquid sample containing a biological material is added dropwise on the reagent layer, so as to develop a color.

## Patentansprüche

1. Trockenanalyseelement, das auf einem transparenten Träger eine Reagenzschicht umfasst, umfassend eine Gelatinedünnschicht, die mindestens ein wasserabsorbierendes Polymer vom Polyacrylattyp enthält, wobei die Reagenzschicht mindestens ein oberflächenaktives Mittel umfasst, wobei das oberflächenaktive Mittel eine Verbindung umfasst, die Silicium enthält.

2. Trockenanalyseelement gemäß Anspruch 1, wobei das wasserabsorbierende Polymer in der Reagenzschicht in einer Menge von nicht mehr als 10 Massen-%, bezogen auf eine Menge an Gelatine pro Einheitsfläche, vorliegt.

3. Trockenanalyseelement gemäß Anspruch 1 oder 2, wobei ein Gelatinegehalt in der Reagenzschicht innerhalb eines Bereichs von 2 bis 100 g/m² liegt.

4. Trockenanalyseelement gemäß Anspruch 4, wobei das oberflächenaktive Mittel in der Reagenzschicht in einer Menge von nicht mehr als 80 Massen-%, bezogen auf eine Menge an Gelatine pro Einheitsfläche, vorliegt.

5. Trockenanalyseelement gemäß einem der Ansprüche 1 bis 4, wobei die Reagenzschicht umfasst: ein Reagenz zum Umwandeln von Pyrophosphat in anorganisches Phosphat und eine Reagenzgruppe zum Bewirken einer Farbreaktion entsprechend einer Menge des umgewandelten anorganischen Phosphats.

6. Trockenanalyseelement gemäß Anspruch 5, wobei die Reagenzschicht umfasst: Xanthosin oder Inosin, anorganische Pyrophosphatase, Purinnucleosidphosphorylase, Xanthinoxidase, Peroxidase und ein Kupplungsmittel.

7. Trockenanalyseelement gemäß Anspruch 6, wobei das Kupplungsmittel ein Farbstoff vom Leukotyp ist.

8. Trockenanalyseelement gemäß Anspruch 5, wobei die Reagenzschicht umfasst: Xanthosin oder Inosin, anorganische Pyrophosphatase, Purinnucleosidphosphorylase, Xanthindehydrogenase, Diaphorase und ein Kupplungsmittel

9. Trockenanalyseelement gemäß Anspruch 8, wobei das Kupplungsmittel ein Farbstoff vom Formazantyp ist.

10. Trockenanalyseelement gemäß einem der Ansprüche 5 bis 9, wobei die Reagenzschicht Magnesiumionen umfasst.

11. Verwendung des Trockenanalyseelements gemäß einem der Ansprüche 1 bis 10 zum Durchführen einer Bestimmung eines biologischen Materials mittels Colorimetrie durch Reflektionsphotometrie oder Transmissionsphotometrie, bei welcher eine flüssige Probe, die ein biologisches Material enthält, tropfenweise auf die Reagenzschicht zugegeben wird, so dass sich eine Farbe entwickelt.

## Revendications

1. Elément d'analyse par voie sèche qui comprend, sur un support transparent, une couche de réactif comprenant un film mince de gélatine contenant au moins un polymère du type polyacrylate absorbant de l'eau, dans lequel la couche de réactif comprend au moins un surfactant, le surfactant comprenant un composé contenant du silicium.

2. Elément d'analyse par voie sèche selon la revendication 1, dans lequel le polymère absorbant de l'eau est présent dans la couche de réactif en une quantité d'au plus 10 % en masse sur la base de la quantité de gélatine par unité de surface.

3. Elément d'analyse par voie sèche selon la revendication 1 ou 2, dans lequel la teneur en gélatine dans la couche de réactif est dans la gamme de 2 à 100 g/m².

4. Elément d'analyse par voie sèche selon la revendication 4, dans lequel, dans la couche de réactif, le surfactant est présent en une quantité d'au plus 80 % en masse sur la base de la quantité de gélatine par unité de surface.

5. Elément d'analyse par voie sèche selon l'une quelconque des revendications 1 à 4, dans lequel la couche de réactif comprend: un réactif pour convertir le pyrophosphate en phosphate inorganique; et un groupe réactif pour réaliser une réaction de coloration dépendante de la quantité du phosphate inorganique converti.

6. Elément d'analyse par voie sèche selon la revendication 5, dans lequel la couche de réactif comprend: la xanthosine ou l'inosine; la pyrophosphatase inorganique; la purine nucléoside phosphorylase; la xanthine oxydase; la peroxydase et un couplant.

7. Elément d'analyse par voie sèche selon la revendication 6, dans lequel le couplant est un colorant du type leuco.

8. Elément d'analyse par voie sèche selon la revendication 5, dans lequel la couche de réactif comprend: la xanthosine ou l'inosine; la pyrophosphatase inorganique; la purine nucléoside phosphorylase; la xanthine déshydrogénase; la diaphorase et un couplant.

9. Elément d'analyse par voie sèche selon la revendication 8, dans lequel le couplant est un colorant du type formazan.

10. Elément d'analyse par voie sèche selon l'une quelconque des revendications 5 à 9, dans lequel la couche de réactif comprend des ions de magnésium.

11. Utilisation de l'élément d'analyse par voie sèche selon l'une quelconque des revendications 1 à 10 pour effectuer l'analyse d'un matériau biologique à l'aide de colorimétrie par photométrie de réflexion ou par photométrie de transmission, dans laquelle un échantillon liquide contenant un matériau biologique est ajouté goutte à goutte sur la couche de réactif pour développer une coloration.
